(19) Europäisches Patentamt

European Patent Office

Office européen des brevets

(11) Numéro de publication : **0 320 428 B1**

(12) **FASCICULE DE BREVET EUROPEEN**

(45) Date de publication du fascicule du brevet :
**05.02.92 Bulletin 92/06**

(21) Numéro de dépôt : **88480095.4**

(22) Date de dépôt : **08.12.88**

(51) Int. Cl.⁵ : **A23L 1/22,** A23P 1/08,
A61F 6/04, A61J 11/00,
A61J 17/00

(54) **Procédé pour l'aromatisation de produits façonnés à base d'élastomère.**

(30) Priorité : **08.12.87 FR 8717204**

(43) Date de publication de la demande :
**14.06.89 Bulletin 89/24**

(45) Mention de la délivrance du brevet :
**05.02.92 Bulletin 92/06**

(84) Etats contractants désignés :
**AT BE CH DE ES FR GB GR IT LI LU NL SE**

(56) Documents cités :
**EP-A- 0 070 719**
**DE-A- 2 037 947**
**US-A- 3 819 838**
**US-A- 3 906 116**
**US-A- 4 276 312**

(73) Titulaire : **DERVIEUX, Dominique**
**Villa Artémise 2ter avenue Henri Barbusse**
**F-06100 Nice (FR)**

Titulaire : **Nouri, Gérard**
**2D allée du Parc des Couvents**
**F-57158 Montigny les Metz (FR)**
Titulaire : **Munger, Jacky**
**3 rue du Haut Poirier**
**F-57000 Metz (FR)**

(72) Inventeur : **DERVIEUX, Dominique**
**Villa Artémise 2ter avenue Henri Barbusse**
**F-06100 Nice (FR)**
Inventeur : **Nouri, Gérard**
**2D allée du Parc des Couvents**
**F-57158 Montigny les Metz (FR)**
Inventeur : **Munger, Jacky**
**3 rue du Haut Poirier**
**F-57000 Metz (FR)**

(74) Mandataire : **Hautier, Jean-Louis**
**Cabinet Hautier Office Méditerranéen de**
**Brevets d'Invention et de Marques 24 rue**
**Masséna**
**F-06000 Nice (FR)**

**Description**

La présente invention a pour objet des compositions pour l'aromatisation de produits façonnés à base d'élastomères et se rapporte aux procédés d'aromatisation de tels produits façonnés par contact avec cette composition ainsi qu'aux conditionnements contenant les produits façonnés susceptibles d'être aromatisés par cette composition juste avant emploi. Par produits formés d'élastomères, on entend des produits tels tétines, préservatifs, etc.

On a tenté, dans le passé, d'aromatiser des produits formés d'élastomères et ces tentatives mises en oeuvre sur la matière première, en général du latex, ont consisté à introduire des composés aromatisés ou arômes dans la masse des élastomères, c'est-à-dire à aromatiser la matière première préalablement au façonnage desdits produits ou bien, en mettant en contact direct des arômes non isolés. L'inconvénient de ces méthodes est qu'elles ont conduit à une moindre résistance de ces élastomères et donc à des risques d'éclatement d'autant plus grands que le produit est fin ; ce risque est par conséquent élevé pour les préservatifs et conduit aux inconvénients que l'on sait.

Il faut encore remarquer que d'autres tentatives ont consisté à mettre des arômes au contact direct des élastomères, mais que ceci a montré que les produits aromatisés vieillissent mal surtout lorsqu'ils sont en contact direct avec des élastomères en présence d'humidité et sous conditionnement contenant de l'air.

La présente invention permet de pallier les inconvénients des procédés connus d'aromatisation de ces élastomères.

Elle se rapporte à des compositions d'aromatisation ne conduisant à aucune dénaturation des élastomères et leur conservant leurs propriétés physiques même à long terme et pour des températures variant sur une large plage. De plus, ces compositions ont des propriétés "alimentaires", c'est-à-dire qu'elles ne sont ni toxiques ni allergènes tant au plan des muqueuses et du tractus digestif, qu'au plan cutané ; elles peuvent être stérilisées, par exemple par irradiation (type β ou autres) et conditionnées en emballages ou conditionnements étanches exempts d'air.

La présente invention a pour objet, à titre de produit industriel nouveau, une composition solide ou liquide de qualité alimentaire, non allergène et non toxique, pour l'aromatisation des produits façonnés formés d'élastomères, caractérisée en ce qu'elle contient, outre un ou plusieurs arômes naturels ou artificiels, un ou plusieurs additifs appartenant au groupe des agents sucrants, édulcorants, renforçateurs du goût, absorbants, colorants, lubrifiants, émulsifiants, stabilisants, épaississants, gélifiants, absorbants, anti-mottants, conservateurs alimentaires, anti-oxydants et autres et qu'elles peuvent être stérilisées, par exemple par irradiation (type β ou autres) et/ou mises dans des conditionnements exempts d'air.

Selon une forme d'exécution de l'invention, la composition est solide et, outre un ou plusieurs arômes naturels ou artificiels, contient au moins un des additifs précités.

Selon une autre forme d'exécution de l'invention, la composition est solide et contient plus de 0% à près de 100% d'un ou plusieurs arômes naturels ou artificiels, 0% à 50% d'agent anti-mottant, 0% à près de 100% d'agent sucrant ou édulcorant, 0% à 20% d'agents renforçateurs du goût, 0% à 20% d'agent colorant, 0% à 30% de conservateurs alimentaires, 0% à 8% d'agent anti-oxydant.

Selon une autre forme encore d'exécution de l'invention, la composition est solide et les arômes naturels ou artificiels des compositions précitées sont micro-encapsulés par atomisation dans un matériau tel que malto-dextrine, gomme arabique, gomme d'acacia, amidon de maïs modifié.

De préférence, ledit matériau d'encapsulation consiste en gomme arabique ou gomme d'acacia.

Cette micro-encapsulation permet une isolation des arômes et, par conséquent, une meilleure stabilité des élastomères.

Selon un mode de réalisation de l'invention, l'agent anti-mottant est un dessicateur alimentaire du type carbonate de magnésium, silice, silice colloïdale, silicates ferriques ou leurs dérivés.

Selon une autre forme d'exécution de l'invention, la composition est liquide et est formée d'une émulsion ou d'une solution aqueuse ou alcoolique d'arômes naturels ou artificiels et de qualité alimentaire. Les arômes peuvent être des extraits naturels totaux ou alcooliques.

Selon un autre mode de réalisation de l'invention, l'agent sucrant est un sucre naturel ou synthétique tel cérulose, glucose, lactose, saccharose, de préférence un sucre naturel extrait de canne à sucre ou de betterave, ou un édulcorant par exemple des édulcorants de synthèse tels saccharine, aspartam, sorbitol, etc. ou des édulcorants naturels tels que suc de réglisse, etc., ou un mélange d'agents sucrants.

Selon un mode de réalisation de l'invention, convenant de préférence aux compositions liquides, au moins un des additifs est de préférence un additif appartenant au groupe des alginates, pectines, carraghénates, gommes, gélatines, glycérine et dérivés, amidons modifiés.

Selon une autre forme d'exécution de l'invention, la composition est liquide et contient 0% à près de 100% d'un ou plusieurs arômes naturels ou artificiels, 0% à près de 100% d'agent émulsifiant, 0% à près de 100% d'agent sucrant ou édulcorant, 0% à 20% d'agents renforçateurs du goût 0% à 20% d'agent colorant, 0% à 30% de conservateurs alimentaires, 0% à 8% (de préférence 0,01%) d'anti-oxydant, le reste étant constitué d'eau ou d'une solution hydroalcoolique à 30°Dé (Degré Celsius) et de qualité alimentaire. De préférence, l'anti-oxydant est employé en une proportion de l'ordre de 0,01%.

Selon un autre mode de réalisation de l'invention, quels que soient les arômes ou additifs, au moins un des additifs appartient au groupe des lubrifiants comestibles d'origine naturelle tel huile neutre par mélange de triglycérides d'acides gras saturés d'origine végétale, de préférence à longueur de chaîne moyenne, ou artificiels, tels huiles ou graisses alimentaires, etc. De préférence :

— d'origine naturelle,

— de faible viscosité : s'étalant facilement sur la peau sans laisser trace de brillant, sans tacher profondément les linges,

— très stable aux températures ambiantes extrêmes et à une forte humidité.

— avec une bonne solubilité et miscibilité à l'alcool éthylique permettant de fixer les huiles essentielles et autres principes actifs volatils et sensibles à l'oxydation, type : arômes d'agrumes : citron, orange, fleur d'orange, mandarine, etc. et mélanges ou d'aromates : menthe, anis, etc. nécessaires en faible proportion.

Il faut cependant éviter que ces arômes ou additifs ne soient des phénols ou leurs dérivés, des antiseptiques à base d'huile, des graisses dérivées du pétrole, de l'essence, du kérosène ainsi que des produits organiques en dérivant, ce qui élimine une grande partie des arômes artificiels.

L'avantage des solutions liquides est, qu'en plus de leur action lubrifiante, elles peuvent présenter un caractère volatil et/ou être complètement absorbées par l'épiderme ou une muqueuse.

Selon un autre mode de réalisation de l'invention, ladite composition est formée d'un mélange d'une composition solide telle que celles qui contiennent des arômes naturels ou artificiels qui peuvent être micro-encapsulés par atomisation de gomme arabique ou d'acacia ou d'amidon de maïs modifié, etc., et d'une composition liquide ou pâteuse contenant des lubrifiants comestibles, naturels ou artificiels, tels les graisses ou huiles alimentaires, ou autres.

L'avantage de ce mélange est l'isolation des arômes dans une huile ou une graisse comestible sous forme de pommade, crème, émulsion fluide, suspension, mélange.

La présente invention a également pour objet les procédés d'aromatisation d'un produit façonné formé d'élastomères consistant à mettre ledit produit au contact de la composition précitée postérieurement à l'obtention du produit façonné et, de préférence, juste avant emploi dudit produit, c'est-à-dire en préparation extemporanée.

Selon une forme d'exécution du procédé de l'invention, l'aromatisation du produit façonné est obtenu par disposition du produit façonné et de ladite composition solide ou liquide dans un même conditionnement unitaire avant scellement.

Selon une autre forme d'exécution du procédé de l'invention, l'aromatisation du produit façonné est obtenu par disposition du produit façonné et de ladite composition dans deux poches distinctes et séparées par au moins une paroi d'un conditionnement unitaire avant scellement, la paroi pouvant être rompue par pression ou retirée partiellement juste avant emploi dudit produit.

De préférence, ladite composition est liquide.

L'invention a également pour objet un conditionnement unitaire scellé contenant le produit façonné à base d'élastomère et la composition liquide ou solide précités qui est isolée du produit façonné à aromatiser. Ladite composition est disposée dans une poche sous vide d'air, susceptible d'éclater par exercice d'une pression externe, cette poche étant donc elle-même disposée dans l'enveloppe formant le conditionnement de façon libre ou plus moins fixe.

Selon une autre forme d'exécution de l'invention, le conditionnement unitaire scellé comporte une ou plusieurs parois médiane(s) plus fragile(s) susceptible(s) d'être rompue(s) par pression ou retirée(s) partiellement, le produit façonné à base d'élastomère et ladite composition étant chacun contenus dans l'une des deux poches distinctes ainsi séparées par au moins une paroi plus fragile. Cette paroi médiane peut elle-même être double et contenir soit le produit façonné ou ladite composition, en l'absence d'air et n'être scellée que partiellement avec le conditionnement unitaire.

Selon une autre forme d'exécution, la paroi fragile telle que précitée séparant le produit façonné et ladite composition, n'est scellée que sur une partie de l'une des faces internes d'une des parois du conditionnement unitaire.

Selon une autre forme encore d'exécution de l'invention, le conditionnement unitaire scellé comporte une seule enveloppe dans laquelle sont contenus, séparés par une couche isolante, le produit façonné à base d'élastomère et un support poreux imprégné d'arômes, la couche isolante étant retirée juste avant emploi et le contact du produit façonné et du support poreux assuré par pression. De la sorte, le liquide absorbé dans le support poreux est exprimé et imbibe le produit façonné.

Le choix des matériaux des conditionnements, enveloppes, parois de séparation, isolants et supports des formes de réalisation précitées sera effectué sur différents critères :

— les matériaux doivent être thermosoudables de façon hermétique ;

— ils doivent permettre la stérilisation du contenu en particulier par irradiation (type ou autres...) ;

— ils permettront éventuellement le vide d'air, ne serait-ce que pour la partie contenant le composé aromatisé ou pour les deux parties ;

— ils seront opaques à la lumière et de qualité "alimentaire", exempt de toute toxicité compatibles chimiquement avec les arômes et additifs ;

— certaines des parois ou doublage des parois peuvent cependant être transparentes ou avoir des fenêtres transparentes permettant de vérifier une ou la bonne conservation des produits et l'absence de passage du composé solide ou liquide dans la poche où se trouve le produit façonné si celui-ci est isolé du composé.

Les dessins ci-joints sont donnés à titre d'exemples indicatifs et non limitatifs. Ils représentent des modes de réalisation préférés selon l'invention. Ils permettront de comprendre aisément l'invention.

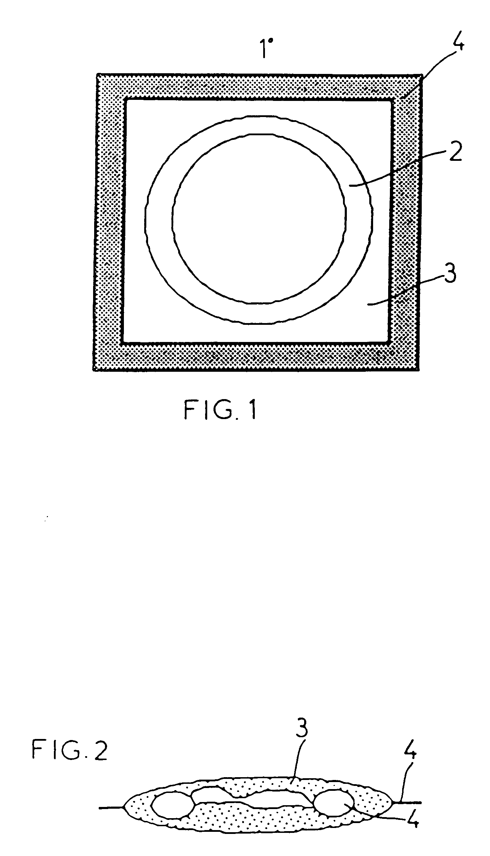

La figure 1 est une vue en plan d'un mode de réalisation où le conditionnement 1 est une poche unique, fermé par thermosoudure sur sa périphérie 4 de deux feuillets thermosoudables, lequel conditionnement contient le préservatif 2 et la poudre ou liquide aromatisé 3.

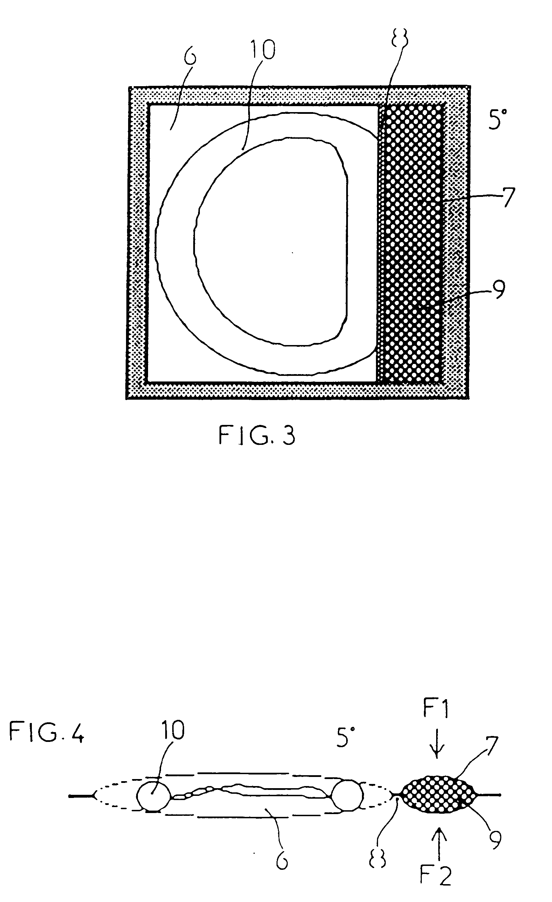

La figure 2 est une vue en coupe longitudinale du conditionnement représenté à la figure 1.

La figure 3 est une vue en plan d'un autre mode de réalisation où le conditionnement 5 est formé par deux poches 6 et 7 séparées par une thermosoudure 8. La poche 6 contient le préservatif 10, l'autre poche 7 contient le liquide aromatisé 9. Une pression exercée selon les flèches F1 et F2 (figure 4) permet de provoquer le contact entre la composition liquide aromatisée 9 et le préservatif 10.

La figure 4 est une vue en coupe du mode de réalisation selon la figure 3.

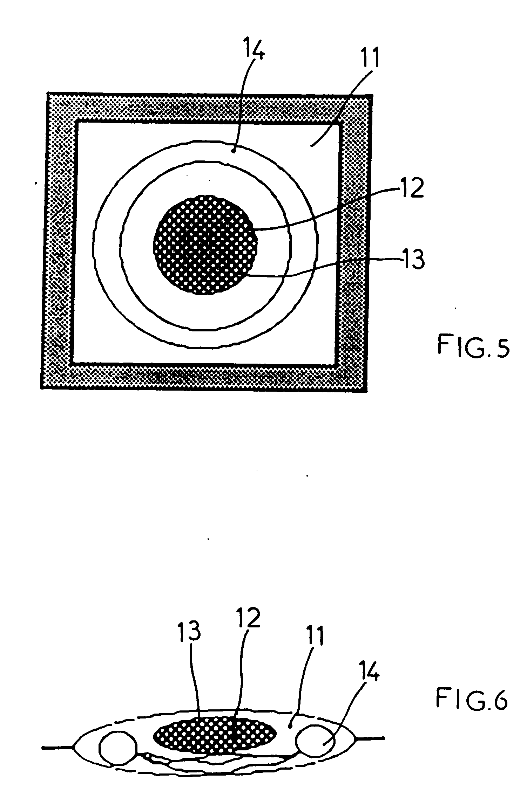

La figure 5 est une vue en plan d'un autre mode de conditionnement formé de deux poches 11 et 12. Une poche 11 contient le préservatif 14 et la poche 12 contient le liquide aromatisé 13, cette poche 12 est facile à rompre par pression.

La figure 6 est une vue en coupe du mode de réalisation selon la figure 5.

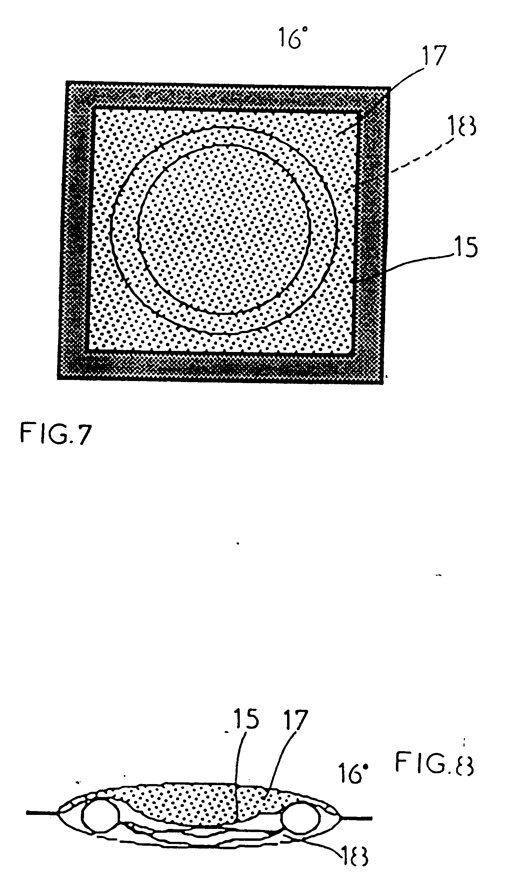

La figure 7 est une vue en plan où les deux poches 17, 18 sont fermées par une cloison médiane 15 qui sépare le conditionnement 16.

La figure 8 est vue en coupe selon le mode de réalisation représenté à la figure 7.

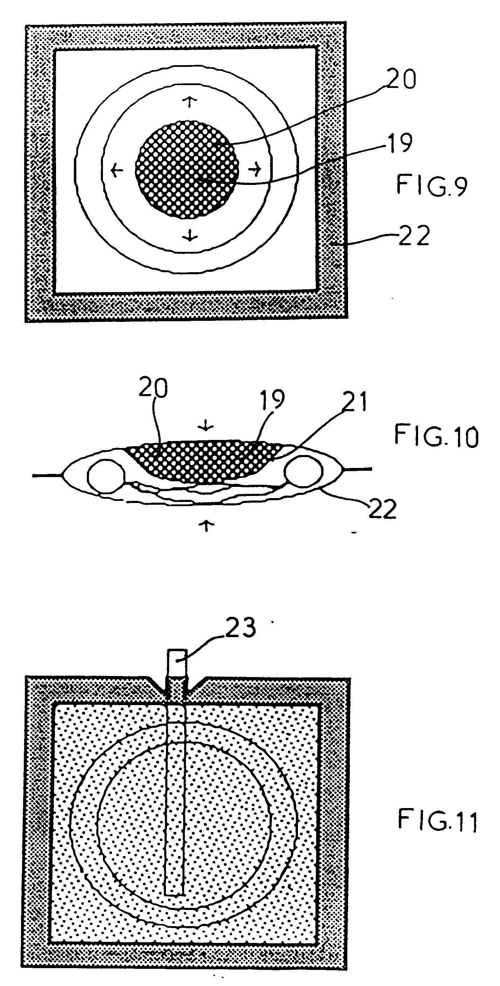

La figure 9 est une vue en plan du conditionnement où la poche hermétique 19 contenant la composition aromatisée 20 est fixée par thermosoudure à la face interne 21 du conditionnement unitaire 22.

La figure 10 est une vue en coupe du mode de réalisation représenté à la figure 9.

La figure 11 est une vue en plan d'un mode de réalisation où la cloison médiane est prévue dans la poche thermosoudée déchirable de l'extérieur. A cet effet, une tirette 23 est prévue pour actionner la déchirure de la cloison interne.

D'autres buts et avantages de l'invention apparaîtront à la lecture de la description suivante et des exemples.

Exemple 1 :

Dans un conditionnement comportant une poche unique fermée par scellement pour être ensuite irradiée par rayon β, on place une tétine, en un élastomère consistant en polybutadiène de qualité alimentaire et une composition solide d'aromatisation formée de :

— 90% d'arômes naturels micro-encapsulés dans de la gomme arabique ;

— 10% d'aérosil.

On constate que le contact direct de la composition avec cette tétine ne dénature pas l'élastomère dont elle est formée ni ne lui fait perdre ses qualités physiques. La conservation pendant 12 mois à température ambiante ou pendant 7 jours à 70°C dans un autoclave ne montre aucune dénaturation ou modification des qualités physiques de la tétine.

Exemple 2 :

On opère comme dans l'exemple 1 en plaçant un préservatif en latex d'hévéa et en utilisant cependant 98% d'arômes naturels micro-encapsulés dans de la gomme d'acacia et 2% de silice colloïdale anti-mottante. On obtient des résultats identiques à ceux qui sont consignés dans l'exemple 1.

Exemple 3 :

Dans un conditionnement comportant une poche unique fermée par scellement, sur sa périphérie, on place une tétine en un élastomère consistant en polybutadiène de qualité alimentaire et une composition liquide d'aromatisation formée de :

— 20% d'une solution aqueuse à 40% d'arômes naturels consistant en un extrait de fruit,

— 30% de carraghénates à 15% d'humidité,

— 49% d'eau,

— 1% de conservateurs.

— stérilisation par irradiation.

On constate que le contact direct de la composition avec cette tétine ne dénature pas l'élastomère dont elle est formée ni ne lui fait perdre ses qualités physiques. La conservation pendant 12 mois à température ambiante ou pendant 7 jours à 70°C dans un autoclave ne montre aucune dénaturation ou modification des qualités physiques de la tétine.

Exemple 4 :

Dans un conditionnement formé d'une enveloppe double fermée par scellement sur sa périphérie, la paroi entre les deux poches étant susceptibles d'être rompue par pression, on introduit dans une poche un préservatif formé d'élastomères d'hévéa et dans l'autre poche une composition liquide d'aromatisation formée de :

— 99% d'arômes alimentaires en solution à 40% dans de l'eau,

— 1% d'un conservateur consistant en sorbate de sodium, le tout étant irradié par rayons stérilisants.

Lorsque l'on souhaite employer le préservatif, on exerce une pression sur le conditionnement pour percer la paroi entre les deux poches et provoquer le contact entre la composition liquide d'aromatisation et le préservatif. On malaxe légèrement le conditionnement préalablement à son ouverture pour assurer un bon contact entre le préservatif et la composition liquide.

## Exemple 5 :

Dans un conditionnement formé d'une enveloppe fermée par scellement sur sa périphérie, la paroi entre les deux poches étant susceptibles d'être rompue par pression, on introduit dans une poche un préservatif en latex d'hévéa et dans l'autre poche une composition liquide d'aromatisation formée de :

— 89% d'eau,

— 10% de saccharose,

— 1% d'arôme artificiel,

— 1% de conservateur consistant en sorbate de sodium stérilisé par irradiation.

Lorsque l'on souhaite employer le préservatif, on exerce une pression sur le conditionnement pour percer la paroi entre les deux poches et provoquer le contact entre la composition d'aromatisation et le préservatif. On presse sur la poche de liquide et malaxe légèrement le conditionnement préalablement à son ouverture pour assurer un bon contact entre le préservatif et la composition solide.

## Exemple 6 :

On opère comme dans l'exemple 5, en remplaçant la composition liquide par une composition pâteuse formée de :

— 50% d'arôme naturel micro-encapsulé dans une gomme d'acacia,

— 0,01% d'un anti-oxydant,

— q.s.p. 100 d'une huile neutre d'origine végétale.

## Exemple 7 :

On imprègne un support poreux de la même composition liquide que dans l'exemple 5. Le support ainsi imprégné et une tétine élastomère sont placés dans une poche unique fermée par scellement sur périphérie. Juste avant emploi, par pression exercée sur l'emballage, la composition est exprimée et imbibe la tétine.

## Exemple 8 :

On utilise la composition formée de :

— un pour mille d'essence de citron,

— q.s.p. 100 d'une huile neutre d'origine végétale : mélange de triglycérides d'acides gras saturés, d'origine, végétale à longueur de chaîne moyenne, que l'on place dans une poche sous vide d'air susceptible d'être rompue par pression, cette poche et le préservatif étant placés dans une enveloppe unique fermée par scellement sur sa périphérie. Une pression exercée sur l'enveloppe permet de produire l'éclatement de la poche interne et l'aromatisation conséquente du préservatif.

## REFERENCES

1. Conditionnement
2. Préservatif
3. Poudre ou liquide aromatisé
4. Thermosoudure
5. Conditionnement
6. Poche
7. Poche
8. Thermosoudure
9. Liquide aromatisé
10. Préservatif
11. Poche
12. Poche
13. Liquide aromatisé
14. Préservatif
15. Cloison médiane
16. Conditionnement
17. Poche
18. Poche
19. Poche hermétique
20. Composition aromatisée
21. Face interne
22. Conditionnement unitaire
23. Tirette
F1-F2. Flèches

## Revendications

1. Procédé d'aromatisation d'un produit façonné formé d'élastomère caractérisé en ce qu'il consiste à mettre au contact dudit produit une composition solide ou liquide de qualité alimentaire, non allergène et non toxique, chimiquement ou physiquement isolée du contact direct avec ledit produit, outre un ou plusieurs arômes naturels ou artificiels contenant un ou plusieurs additifs appartenant au groupe formé par les agents micro-encapsulants, sucrants, édulcorants, renforçateurs du goût, absorbants, colorants, lubrifiants, émulsifiants, stabilisants, épaississants, gélifiants, absorbants, anti-mottants, conservateurs alimentaires, anti-oxydants.

2. Procédé d'aromatisation selon la revendication 1, caractérisé en ce que la composition est solide et, outre un ou plusieurs arômes naturels ou artificiels, contient au moins un des additifs précités.

3. Procédé d'aromatisation selon la revendication 2, caractérisé en ce que la composition contient 0% à près de 100% d'un ou plusieurs arômes naturels ou artificiels, 0% à 50% d'agent anti-mottant, 0% à près de 100% d'agent sucrant ou édulcorant, 0% à 20% d'agents renforçateurs du goût, 0% à 20% d'agent colorant, 0% à 30% de conservateurs alimentaires, 0% à 8% d'agent anti-oxydant.

4. Procédé d'aromatisation selon la revendication 2 ou 3, caractérisé en ce que la composition contient un ou plusieurs arômes naturels ou artificiels micro-encapsulés obtenus par atomisation dans un matériau tel malto-dextrine, gomme arabique, gomme d'acacia, amidon de maïs modifié.

5. Procédé d'aromatisation selon la revendication 4, caractérisé en ce que le matériau d'encapsulation consiste en gomme arabique ou gomme d'acacia.

6. Procédé d'aromatisation selon une quelconque des revendications 1 à 5, caractérisé en ce que l'agent anti-mottant est un dessicateur alimentaire du type carbonate de magnésium, silice, silice colloïdale, silicates ferriques ou leurs dérivés.

7. Procédé d'aromatisation selon la revendication 1, caractérisé en ce que la composition est liquide et est formée d'une émulsion ou d'une solution aqueuse ou alcoolique d'un ou plusieurs arômes naturels ou artificiels de qualité alimentaire.

8. Procédé d'aromatisation selon la revendication 6, caractérisé en ce que la composition est liquide et contient 0% à près de 100% d'un ou plusieurs arômes naturels ou artificiels, 0% à près de 100% d'agent émulsifiant, 0% à près de 100% d'agent sucrant ou édulcorant, 0% à 20% d'agents renforçateurs du goût, 0% à 20% d'agent colorant 0% à 30% de conservateurs alimentaires, 0% à 8% d'agent anti-oxydant, le reste étant constitué d'eau ou d'une solution hydro-alcoolique à 30°Dé et de qualité alimentaire.

9. Procédé d'aromatisation selon une quelconque des revendications 1 à 8, caractérisé en ce que l'agent sucrant est un sucre naturel ou artificiel tel cérulose, glucose, lactose, saccharose, ou un édulcorant.

10. Procédé d'aromatisation selon une quelconque des revendications 1 à 8, caractérisé en ce qu'au moins un des additifs appartient au groupe des alginates, pectines, carraghénates, gommes, gélatines, glycérine et dérivés, amidons modifiés.

11. Procédé d'aromatisation selon une quelconque des revendications 1 à 9, caractérisé en ce qu'au moins un des additifs appartient au groupe des lubrifiants naturels ou artificiels, comestibles, tels huiles ou graisses alimentaires, cet additif étant éventuellement additionné d'un anti-oxydant.

12. Procédé d'aromatisation selon la revendication 11 caractérisé en ce que la composition est associée à une huile neutre d'origine végétale constituée d'un mélange de triglycérides d'acides gras saturés, de préférence à longueur de chaîne moyenne, de faible viscosité, très stable, de bonne solubilité et miscibilité avec l'alcool éthylique permettant de fixer des huiles essentielles et un ou plusieurs arômes d'agrumes tels citron, orange, fleur d'oranger, mandarine, ou d'aromates tels menthe, anis, miscibles et ajoutés en faible proportion.

13. Procédé d'aromatisation selon l'une quelconque des revendications 1 à 12, caractérisé en ce que la composition est obtenue par mélange d'une composition solide contenant des arômes naturels ou artificiels micro-encapsulés par atomisation de gomme arabique ou d'acacia ou d'amidon de maïs modifié, avec une composition liquide ou pâteuse contenant des lubrifiants comestibles, naturels ou artificiels, tels les graisses ou huiles alimentaires, ou autres.

14. Procédé d'aromatisation selon la revendication 11 caractérisé en ce que la composition est obtenue par mélange d'un silicone aromatisé avec une micro-encapsulation des parfums dans de la gomme arabique.

15. Procédé d'aromatisation d'un produit façonné formé d'élastomères à l'aide de la composition selon une quelconque des revendications 1 à 14, caractérisé en ce qu'il consiste à mettre ledit produit au contact de ladite composition juste avant emploi dudit produit.

16. Procédé d'aromatisation selon la revendication 15, caractérisé en ce que l'aromatisation du produit façonné est obtenue par disposition du produit façonné et de ladite composition dans un même conditionnement unitaire avant scellement.

17. Procédé d'aromatisation selon la revendication 16, caractérisé en ce que l'aromatisation du produit façonné est obtenue par disposition du produit façonné et de ladite composition dans deux poches distinctes et séparées par une paroi d'un conditionnement unitaire avant scellement, la paroi étant rompue par pression ou retirée partiellement juste avant emploi dudit produit.

18. Conditionnement unitaire scellé obtenu à l'aide du procédé selon l'une quelconque des revendications 1 à 13 caractérisé en ce que ladite composition est isolée du produit façonné à aromatiser.

19. Conditionnement selon la revendication 18, caractérisé en ce qu'il contient dans une même poche le produit façonné à base d'élastomère et ladite composition.

20. Conditionnement selon la revendication 18, caractérisé en ce qu'il comporte ladite composition à l'état liquide ou solide disposée dans une poche susceptible d'éclater par exercice d'une pression externe, elle-même disposée dans la poche formant le conditionnement.

21. Conditionnement selon le procédé des revendications 1 à 17, caractérisé en ce qu'il comporte deux poches distinctes séparées par une paroi susceptible d'être rompue par pression ou retirée au moins partiellement, le produit façonné à base d'élastomère et ladite composition étant chacun contenus dans l'une des poches.

22. Conditionnement obtenu le procédé des revendications 1 à 17, caractérisé en ce qu'il comporte une seule poche dans laquelle sont contenus, séparés par une couche isolante, le produit façonné à base d'élastomère et un support poreux imprégné d'arômes, la couche isolante étant rompue ou retirée au moins partiellement, juste avant emploi et le contact du produit façonné et du support poreux étant assuré par pression de sorte que le liquide absorbé dans le support poreux soit exprimé et imbibe le produit façonné.

23. Conditionnement selon l'une quelconque des revendications 18 à 22 caractérisé en ce qu'une ou plusieurs des parois ou doublage des parois peuvent être en matière transparente ou comporter une ou plusieurs fenêtres transparentes pour permettre de vérifier la conservation des produits et l'absence de passage de la composition aromatisée vers la poche où se trouve le produit façonné.

24. Conditionnement selon les revendications 18 à 23 caractérisé en ce que les matériaux utilisés pour le conditionnement sont thermosoudables de façon hermétique, opaque à la lumière, de qualité "alimentaire" et pouvant permettre la stérilisation du contenu.

25. Conditionnement selon l'une quelconque des revendications 17 à 22 caractérisé en ce que le produit façonné à base d'élastomère est un préservatif.

**Patentansprüche**

1. Verfahren zur Aromatisierung eines bearbeiteten, aus Elastomer gebildeten Produkts, dadurch gekennzeichnet, daß es darin besteht, in Kontakt mit diesem Produkt eine feste oder flüssige, nicht allergene und nicht giftige, chemisch oder physikalisch vom direkten Kontakt mit dem Produkt isolierte Zusammensetzung von Nahrungsmittelqualität zu bringen, die außer einem oder mehreren natürlichen oder künstlichen Aromen einen oder mehrere Zusätze enthält, die zur Gruppe gehören, die durch die Mikroverkapselungs-, Zuckerungs-, Süßungs-, Geschmacksverstärkungs-, Absorptions-, Färbe-, Schmier-, Emulgier-, Stabilisier-, Verdickungs-, Gelier-, Absorptions-, Klumpenverhinderungs-, Nahrungsmittelkonservier-, Antioxidationsmittel gebildet wird.

2. Aromatisierungsverfahren nach dem Anspruch 1, dadurch gekennzeichnet, daß die Zusammensetzung fest ist und außer einem oder mehreren natürlichen oder künstlichen Aromen wenigstens einen der vorgenannten Zusätze enthält.

3. Aromatisierungsverfahren nach dem Anspruch 2, dadurch gekennzeichnet, daß die Zusammensetzung 0% bis nahe 100% eines oder mehrerer natürlicher oder künstlicher Aromen, 0% bis 50% eines Klumpenverhinderungsmittels, 0% bis nahe 100% eines Zuckerungs- oder Süßungsmittels, 0% bis 20% Geschmacksverstärkungsmittel, 0% bis 20% eines Färbemittels, 0% bis 30% Nahrungsmittelkonserviermittel, 0% bis 8% eines Antioxidationsmittels enthält.

4. Aromatisierungsverfahren nach dem Anspruch 2 oder 3, dadurch gekennzeichnet, daß die Zusammensetzung ein oder mehrere natürliche oder künstliche, mikroverkapselte Aromen enthält, die durch Zerstäubung in einem Material wie Maltodextrin, Gummi arabicum, Akaziengummi, modifiziertem Maisstärkemehl erhalten wurden.

5. Aromatisierungsverfahren nach dem Anspruch 4, dadurch gekennzeichnet, daß das Verkapselungsmaterial aus einem Gummi arabicum oder Akaziengummi besteht.

6. Aromatisierungsverfahren nach irgend einem der Ansprüche 1 bis 5, dadurch gekennzeichnet, daß das Klumpenverhinderungsmittel ein Nahrungstrockenmittel des Typs Magnesiumcarbonat, Siliciumdioxid, kolloidales Siliciumdioxid, Eisen(III)-Silikate oder deren Derivate ist.

7. Aromatisierungsverfahren nach dem Anspruch 1, dadurch gekennzeichnet, daß die Zusammensetzung flüssig ist und aus einer wäßrigen oder alkoholischen Emulsion oder Lösung eines oder mehrerer natürlicher oder künstlicher Aromen von Nahrungsmittelqualität gebildet wird.

8. Aromatisierungsverfahren nach dem Anspruch 7, dadurch gekennzeichnet, daß die Zusammensetzung flüssig ist und 0% bis nahe 100% eines oder mehrerer natürlicher oder künstlicher Aromen, 0% bis nahe 100% eines Emulgiermittels, 0% bis nahe 100% eines Zuckerungs- oder Süßungsmittels, 0% bis 20% Geschmacksverstärkungsmittel, 0% bis 20% eines Färbemittels, 0% bis 30% Nahrungsmittelkonserviermittel, 0% bis 8% eines Antioxidationsmittels enthält, wobei der Rest aus Wasser oder einer Wasser-Alkohol-Lösung mit 30°Dé und von Nahrungsmittelqualität besteht.

9. Aromatisierungsverfahren nach irgend einem der Ansprüche 1 bis 8, dadurch gekennzeichnet, daß das Zuckerungsmittel ein natürlicher oder künstlicher Zucker wie Cerulose, Glucose, Lactose, Saccharose oder ein Süßungsmittel ist.

10. Aromatisierungsverfahren nach irgend einem der Ansprüche 1 bis 8, dadurch gekennzeichnet, daß wenigstens einer der Zusätze zur Gruppe der Alginate, Pektine, Karragenate, Gummis, Gelatine, Glycerin und Abkömmlinge, modifizierte Stärkemehle gehört.

11. Aromatisierungsverfahren nach irgend einem der Ansprüche 1 bis 9, dadurch gekennzeichnet, daß wenigstens einer der Zusätze zur Gruppe der eßbaren natürlichen oder künstlichen Schmiermittel, wie Nahrungsmittelöle oder -fette, gehört, welchem Zusatz ggf. ein Antioxidationsmittel zugesetzt wird.

12. Aromatisierungsverfahren nach dem Anspruch 11, dadurch gekennzeichnet, daß die Zusammensetzung mit einem neutralen Öl pflanzlichen Ursprungs verbunden wird, das aus einem Gemisch von Triglyceriden von gesättigten Fettsäuren, vorzugsweise mit mittlerer Kettenlänge, geringer Viskosität, hoher Stabilität, guter Lösbarkeit und Mischbarkeit mit Ethylalkohol besteht, wodurch ermöglicht wird, ätherische Öle und ein oder mehrere Aromen von Zitrusfrüchten, wie z.B. Zitrone, Orange, Orangenblüte, Mandarine, oder aromatische Substanzen, wie z.B. Menthe, Anis, die mischbar sind und in geringem Anteil zugesetzt werden, festzulegen.

13. Aromatisierungsverfahren nach irgend einem der Ansprüche 1 bis 12, dadurch gekennzeichnet, daß die Zusammensetzung durch Vermischen einer festen Zusammensetzung, die natürliche oder künstliche Aromen, die durch Zerstäubung von Gummi arabicum oder Akaziengummi oder modifiziertem Maisstärkemehl mikroverkapselt sind, enthält, mit einer flüssigen oder pastösen Zusammensetzung erhalten wird, die natürliche oder künstliche eßbare Schmiermittel, wie z.B. Nahrungsfette oder -öle, oder andere enthält.

14. Aromatisierungsverfahren nach dem Anspruch 11, dadurch gekennzeichnet, daß die Zusammensetzung durch Vermischen eines aromatisierten Silicons mit einer Mikroverkapselung der Parfums in Gummi arabicum erhalten wird.

15. Verfahren zur Aromatisierung eines bearbeiteten, aus Elastomer gebildeten Produkts mit Hilfe der Zusammensetzung nach irgend einem der Ansprüche 1 bis 14, dadurch gekennzeichnet, daß es darin besteht, das Produkt gerade vor Verwendung dieses Produkts in Kontakt mit dieser Zusammensetzung zu bringen.

16. Aromatisierungsverfahren nach dem Anspruch 15, dadurch gekennzeichnet, daß die Aromatisierung des bearbeiteten Produkts durch Anordnung des bearbeiteten Produkts und der Zusammensetzung in einer selben Einheitsverpackung vor dem Versiegeln erhalten wird.

17. Aromatisierungsverfahren nach dem Anspruch 16, dadurch gekennzeichnet, daß die Aromatisierung des bearbeiteten Produkts durch Anordnung des bearbeiteten Produkts und der Zusammensetzung in zwei besonderen und durch eine Wand getrennten Taschen einer Einheitsverpackung vor dem Versiegeln erhalten wird, welche Wand direkt vor Verwendung des Produkts durch Druck zerbrochen oder teilweise zurückgezogen wird.

18. Mit Hilfe des Verfahrens nach irgend einem der Ansprüche 1 bis 13 erhaltene, versiegelte Einheitsverpackung, dadurch gekennzeichnet, daß die Zusammensetzung von dem zu aromatisierenden bearbeiteten Produkt isoliert ist.

19. Verpackung nach dem Anspruch 18, dadurch gekennzeichnet, daß sie in ein und derselben Tasche das bearbeitete Produkt auf Basis eines Elastomers und die Zusammensetzung enthält.

20. Verpackung nach dem Anspruch 18, dadurch gekennzeichnet, daß sie die Zusammensetzung im flüssigen oder festen Zustand enthält, die in einer durch Einwirkung eines äußeren Drucks zerplatzbaren Tasche angeordnet ist, die selbst in der die Verpackung bildenden Tasche angeordnet ist.

21. Verpackung nach dem Verfahren der Ansprüche 1 bis 17, dadurch gekennzeichnet, daß sie zwei durch eine Wand getrennte besondere Taschen aufweist, welche Wand durch Druck zerbrochen oder wenigstens teilweise zurückgezogen werden kann, wobei das bearbeitete Produkt auf Elastomerbasis und die Zusammensetzung jeweils in einer der Taschen enthalten sind.

22. Nach dem Verfahren der Ansprüche 1 bis 17 erhaltene Verpackung, dadurch gekennzeichnet, daß sie eine einzige Tasche aufweist, in der, durch eine Isolierschicht getrennt, das verarbeitete Produkt auf Elastomerbasis und ein mit Aromen imprägnierter poröser Träger enthalten sind, wobei die Isolierschicht direkt vor Verwendung zerbrochen oder wenigstens teilweise entfernt wird und der Kontakt des verarbeiteten Produkts und des porösen Trägers durch Druck derart gesichert wird, daß die im porösen Träger absorbierte Flüssigkeit ausgedrückt wird und das verarbeitete Produkt tränkt.

23. Verpackung nach irgend einem der Ansprüche 18 bis 22, dadurch gekennzeichnet, daß eine oder mehrere der Wände oder Verdoppelung der Wände aus transparentem Material sein oder ein oder mehrere transparente Fenster aufweisen können, um zu ermöglichen, den Erhaltungszustand der Produkte und die Abwesenheit eines Eindringens der aromatisierten Zusammensetzung zur Tasche zu überprüfen, wo sich das verarbeitete Produkt befindet.

24. Verpackung nach den Ansprüchen 18 bis 23, dadurch gekennzeichnet, daß die für die Verpackung verwendeten Materialien in hermetischer und lichtundurchlässiger Weise mit "Nahrungsmittel"-Qualität wärmeverschweißbar sind und die Sterilisierung des Inhalts zulassen können.

25. Verpackung nach irgend einem der Ansprüche 17 bis 22, dadurch gekennzeichnet, daß das verarbeitete Produkt auf Elastomerbasis ein Präservativ ist.

**Claims**

1. A method for aromatizing a processed product formed of an elastomer, **characterized** in that it consists in bringing into contact with this product a solid or liquid, non-allergenic and non-toxic composition having foodstuff quality and insulated chemically or physically from direct contact with the product, which composition contains in addition to one or several natural or artificial aromas one or several additions of the group formed of the microencapsulating, sugaring, sweetening, taste intensifying, absorption, couloring, lubricating, emulsifying, stabilizing, thickening, gelating, absorption, anti-clotting, foodstuff preserving, antioxidization agents

2. The aromatizing method according to claim 1, characterized in that the composition is solid and in that, in addition to one or several natural or synthetic aromas, it contains at least one of the above additions.

3. The aromatizing method according to claim 2, characterized in that the composition contains 0% to nearly 100% of one or several natural or synthetic aromas, 0% to 50% of an anti-clotting agent, 0% to nearly 100% of a sugaring or sweetening agent, 0% to 20% of a taste intensifying agent, 0% to 20% of a colouring agent, 0% to 30% of a foodstuff preserving agent, 0% to 8% of an antioxidization agent.

4. The aromatizing method according to claim 2 or claim 3, characterized in that the composition contains one or several natural or synthetic, micro-encapsuled aromas which were obtained in a material such as maltodextrin, gum arabic, acacia gum, modified corn starch flour through atomization.

5. The aromatizing method according to claim 4, characterized in that the encapsulating material consists of gum arabic or acacia gum.

6. The aromatizing method according to any of claims 1 to 5, characterized in that the anti-clotting agent is a foodstuff drying agent of the type magnesium carbonate, silicon dioxide, a colloidal silicon dioxide, iron(III)-silicates or the derivatives thereof.

7. The aromatizing method according to claim 1, characterized in that the composition is liquid and is formed of an aqueous or alcoholic emulsion or solution of one or several natural or artifical aromas having foodstuff quality.

8. The aromatizing method according to claim 7, characterized in that, the composition is liquid and contains 0% to nearly 100% of one or several natural or synthetic aromas, 0% to nearly 100% of an emulsifying agent, 0% to nearly 100% of a sugaring or sweetening agent, 0% to 20% of a taste intensifying agent, 0% to 20% of a colouring agent, 0% to 30% of a foodstuff preservation agent, 0% to 8% of an antioxidation agent, balance water or a water-alcohol solution with 30°Dé and having foodstuff quality.

9. The aromatizing method according to any of claims 1 to 8, characterized in that the sugaring agent is a natural or synthetic sugar such as cerulose, glucose, lactose, saccharose or a sweetening agent.

10. The aromatizing method according to any of claims 1 to 8, characterized in that at least one of the additions belongs to the group comprising alginates, pectines, carragenates, gums, gelatins, glycerols and derivatives, modified starch flours.

11. The aromatizing method according to any of claims 1 to 9 characterized in that at least one of the additions belongs to the group of the edible natural or synthetic lubricants, such as foodstuff oils or foodstuff fats, to which addition an antioxidization agent is optionally added.

12. The aromatizing method according to claim 11, characterized in that the composition is associated to (combined with) a neutral oil of plant origin consisting of a mixture of triglycerides of saturated fatty acids, preferably having a mean chain length, a low viscosity, a high stability, a good solubility and misceability with ethyl alcohol, thereby facilitating fixation of ethereal oils and one or several aromas of citrus fruit, for example lemon, orange, orange blossom, mandarin, or aromatic substances such as mint, anise, which are miscible and added in small amounts.

13. The aromatizing method according to any of claims 1 to 12, characterized in that the composition is obtained by mixing a solid composition containing natural or artificial aromas microencapsuled through atomization of gum arabic or acacia gum or modified corn starch flour with a liquid or pasty composition containing natural or synthetic edible lubricants, for example foodstuff fats or foodstuff oils ; or others.

14. The aromatizing method according to claim 11, characterized in that the composition is obtained through mixing an aromatized silicon with a microencapsulation of the perfumes in gum arabic.

15. Method for aromatizing a processed product formed from an elastomer by means of the composition according to any of claims 1 to 14, characterized in that it consists in contacting the product with this composition immediately before use of this product.

16. The aromatizing method according to claim 15, characterized in that aromatizing of the processed product is ensured through placing the processed product and the composition in one and the same uniform (standard) package prior to sealing.

17. The aromatizing method according to claim 16, characterized in that atomization of the processed product is obtained through disposing, prior to sealing, the processed product and the composition in two special pockets of a standard package, which pockets are separated by a wall which, immediately prior to use of the product, is broken by applying pressure, or is partly pulled back.

18. A sealed standard package obtained by means of the method according to any one of claims 1 to 13, characterized in that the composition is isolated from the product to be aromatised.

19. The package according to claim 18, characterized in that it contains in one and the same pocket the processed product on the basis of an elastomer and the composition.

20. The package according to claim 18, characterized in that it contains the composition in the liquid or in the solid state, the composition being disposed in a pocket designed to burst as an external pressure is applied, which pocket itself is disposed in the pocket forming the package.

21. The package according to the method of claims 1 to 17, characterized in that it comprises two special pockets separated by a wall, which wall can be broken by applying pressure or at least be partially pulled back, with the processed product on an elastomer basis and the composition each being contained in one of the pockets.

22. The package obtained according to the method of claims 1 to 17, characterized in that it comprises one pocket only in which, separated by an isolating layer, the processed elastomer basis product and a porous carrier impregnated with aromas are contained, with the isolating layer being broken immediately before use or removed at least partially, and contact of the processed product and of the porous carrier being ensured by the application of pressure such that the liquid absorbed in the porous carrier is squeezed out, imbibing the processed product.

23. The package according to any of claims 18 to 22, characterized in that one or several of the walls or doublings of the walls may be of a transparent material or comprise one or several transparent windows to facilitate verifying the conservation state of the products and the absence of intrusion of the aromatized composition into the pocket where the processed product is disposed.

24. The package according to claims 18 to 23, characterized in that the materials used for the package are apt for heat-welding, opaque to light, have foodstuff quality and permit sterilization of the content.

25. The package according to any of claims 17 to 22, characterized in that the processed product on elastomer basis is a preservative.

FIG. 1

FIG. 2

FIG. 3

FIG. 4

FIG. 5

FIG.6

16°

17

18

15

FIG.7

15  17

16°  FIG.8

18

FIG.9

20

19

22

FIG.10

20

19

21

22

23

FIG.11